# EUROPEAN PATENT APPLICATION

(11) **EP 2 862 547 A1**
(43) Date of publication of application: **22.04.2015**
(21) Application number: 14167197.4
(22) Date of filing: 06.05.2014
(51) Int. Cl.: A61F 5/01

(54) **Lower limb orthosis and the method to control the pressure exerted by the orthosis on the limb**

(30) Priority: 16.10.2013 PL 40566313
(71) Applicant: MDH Sp. z o.o., 90-349 Poland (PL)
(72) Inventor: Baczynski, Tadeusz, 95-070 Aleksandrów Lódzki (PL)
(74) Representative: Rumpel, Alicja

(57) **Abstract**

The lower limb orthosis containing the rigid, non-deformable, at least bipartite casing consisting of the posterior part enclosing the back part of the calf and the bottom part of the foot and of the tibia part enclosing tibia and the upper part of the foot, both parts connected with self-attaching straps, connected in a detachable way with the casing, a pneumatic cuff located in the casing, and a pumping system connected to the pneumatic cuff with a hose; in which the pumping system (4) is connected with the control module (10) monitoring the functioning of the z pumping system (4) and at least one pressure sensor (5).

The method to control the pressure exerted by the orthosis on the limb consisting in that the limb is placed in the pneumatic cuff (3) of the ankle joint orthosis and closed in the non-deformable casing (1) and (2) with the self-attaching straps (8), after which the power supply of the pumping system (4) is switched on and the air is pumped by the pumping system (4) to the compartment of the pneumatic cuff (3) via the pressure sensor (5) in at least 10 sec cycles, and on exceeding the pressure of 25 mmHg it emits a warning signal, and on reaching the pressure of 29 mmHg the control module disconnects the power supply of the pumping system emits the continuous warning signal

## Description

The object of the invention is the orthosis of the lower limb, in particular of the ankle joint, with the pneumatic system for supporting the limb, and the method to control the pressure exerted by the orthosis on the limb.

Orthoses of various types are known are known and widely used in the injury prevention and post-injury rehabilitation; their primary task is to stiffen and/or block the joint which lost partially its functionality as the result of an injury. The loss of functionality is most often the consequence of the injured ligaments or muscle attachments; the effects include both pain syndromes and impairment of the defence mechanisms protecting the joint from the uncontrolled relocation of the individual parts of the skeleton forming the joint.

Orthoses used nowadays are the alternative for plaster casts used in the previous years and allow the patient rehabilitation almost immediately after the occurrence of an injury. Depending on the injured area, orthoses with mobile kinematic systems are used for example for the knee joint, and immobilising orthoses are used for example in case of ankle joints.

As regards their functionality, the latter resemble the commonly used plaster casts; however they enable the temporary removal of the orthosis for the time of hygienic or rehabilitation treatments.

The WO0189410 patent specification presents the ankle joint orthosis with a rigid casing and soft insert adapted to the anatomy of the joint, fixed to the casing with straps.

There are also ankle joint orthoses in which a soft inlay is made as a cushion filled with gas (usually air). The example of such solution is the orthosis described in the US20060229541 patent application where the rigid casing is equipped with at least one airtight chamber filled with air with the use of an external, detachable pump.

In turn, the specification of the US20050145256 patent application discloses the construction of the orthosis in which pneumatic chambers do not enclose evenly the area of the joint but are arranged according to its anatomy. The pumping of chambers takes place via the external pump, and each chamber (right / left) has a separate valve.

The construction of the pneumatic inserts of orthoses also varies greatly. The patent US5496262 discloses the construction of pneumatic compartments which according to patent specification may be used in medical and orthopaedic devices and which uses the system of fluid restrictors controlling the pressure. The air is allowed to the compartments by a pulsing air pump through the system of hoses and valves where these valves are one-way check-valves scaled for the blood pressure and atmospheric pressure.

The construction of pneumatic chambers disclosed in the US20040092853 application is slightly different. The object of the disclosure is a pneumatic splint serving the immobilisation of a fractured or sprained extremity, in which the splint member is associated with an inflatable air-bag disposed, in use, between the splint member and the limb and equipped with a valve; the valve is a safety valve by which air is released from within the air-bag if the pressure of air within the air-bag exceeds a predetermined threshold of a pressure safe for the limb.

There are also pneumatic cushions integrated with pumps, like for example the one disclosed in the application US5865166 which can be employed in the rehabilitation or emergency devices and where the construction comprises a pump and a bleed valve. The pump is operated manually (pressed) until the reduction valve opens.

The already known solutions in which pneumatic chambers are applied significantly improve the stability of the joint area and the comfort of using the orthosis; however the valves applied (usually reduction valves) are scaled mechanically with the rigidness of the restricting spring selected for the assumed pressure. The flexible elements wear out quickly which usually results in poorer stabilisation of the joint and incomplete inflation of the cushions of orthosis. In turn, equipping the orthosis with manual pumps operated by the user results in the overexertion of the impaired limb as the user usually leans on this limb while filling the orthosis in. For these reasons it would be advisable to develop the orthosis which would ensure optimum stabilisation for the limb and at the same time the proper level of compression of the soft tissues in the injured area without compromising the blood supply for the stabilised area. Such conditions are ensured by the lower limb orthosis according to the invention.

The lower limb orthosis according to the invention consists of the rigid, non-deformable, at least bipartite casing connected in a detachable way with a pneumatic cuff located in the casing and also of the pumping system connected to the pneumatic cuff with a hose and quipped with a control module which monitors the functioning of the pumping system, and at least one pressure sensor. Depending on the example of realisation, the pumping system has the form of an electric or manual pump. The control module contains a microprocessor control unit connected to the pressure sensor, pumping system and pressure relief valve. The pressure sensor is a differential sensor with a range at least up to 75 mmHg. In case of the pumping system equipped with an electric pump it is advisable to locate the pumping system at the same printed board as the microprocessor control unit. From the control module to the surface of the casing runs at least one device signalling the exceeded maximum pressure; it is advisable to give the signalling device a form of at least visual signalling device, more favourably a visual and acoustic signalling device; the pressure at which the visual and/or acoustic signal is generated does not exceed 30 mmHg.

The non-deformable casing is bipartite and consists of the posterior part enclosing the back part of the calf and the bottom part of the foot and of the tibia part enclosing tibia and the upper part of the foot. Both parts are mutually connected, most favourably with at least two self-attaching straps. The parts of the casing stabilise the pneumatic cuff located between them.

The pneumatic cuff is built of at least two layers forming the airtight compartment; it is advisable to cover the external surface of these layers with soft fabric. The airtight compartment is equipped with the stem for connecting it to the pumping system and most favourably with a safety valve. The pneumatic cuff is adapted to the anatomic shape of the joint; it is advisable to provide it with an opening at least for the heel. Most favourably the airtight compartment should not enclose the entire pneumatic cuff but only the immediate area of the joint and the remaining part of the pneumatic cuff is padded with a soft foam.

The method to control the pressure exerted by the orthosis on the limb consists in that when the limb is placed in the pneumatic cuff of the ankle joint orthosis and closed in the non-deformable casing with the self-attaching straps, the pumping system power supply is switched on. The air is pumped by the pumping system to the compartment of the pneumatic cuff via the pressure sensor in at least 10 sec cycles where each cycle is launched by the subsequent turning on of the power supply of the pumping system. During pumping the control module analyses the pressure in the compartment and when the pressure of 25 mmHg is exceeded, it emits a warning signal. Most favourably, on reaching the pressure of 29 mmHg the control module should disconnect the power supply of the pumping system and emit the continuous warning signal. It is also favourable if in case of reaching the pressure exceeding 29 mmHg the control module opens the pressure relief valve until the pressure drops to 25 mmHg. It is also favourable if in case of any defect of the control unit or pressure sensor, on exceeding the pressure of 30 mmHg the safety valve opens and reduces the pressure to at least 25 mmHg.

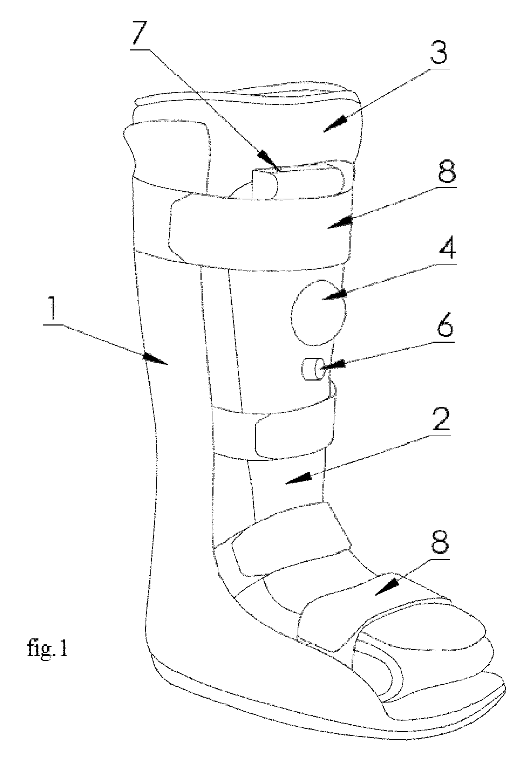

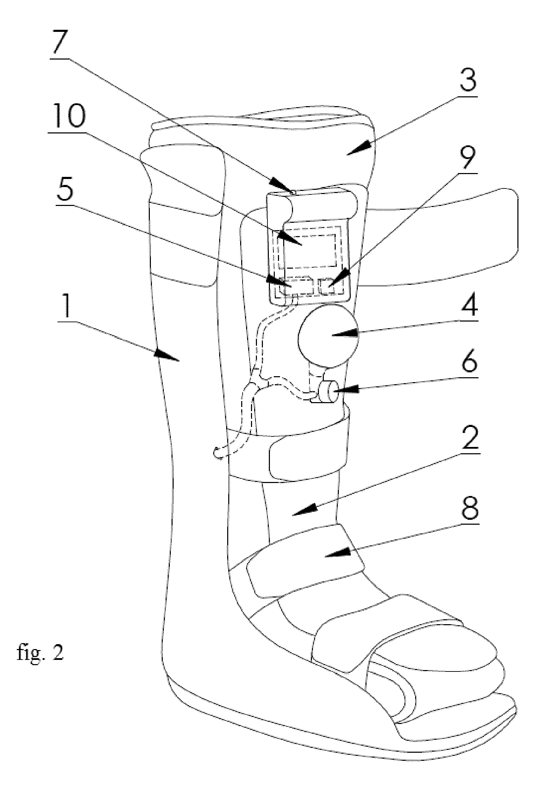

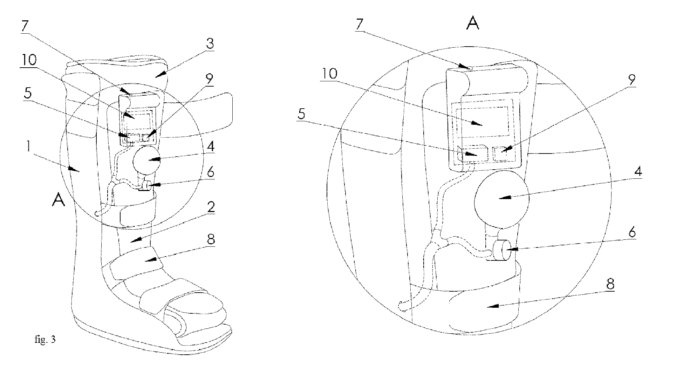

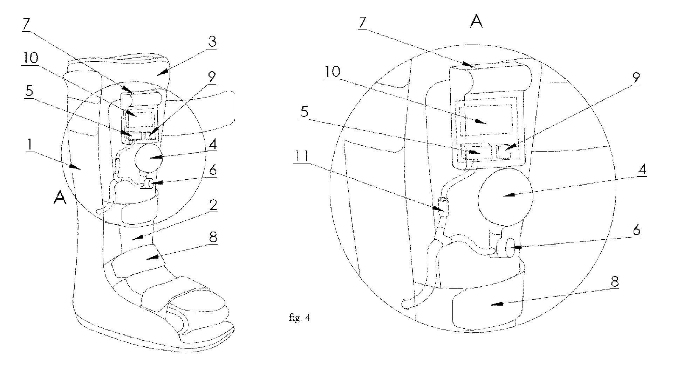

The lower limb orthosis according to the invention, applying the method to control the pressure exerted by the orthosis on the limb according to the invention, is presented in the drawings where the fig. 1 presents the general view of the orthosis according to the invention, the fig. 2 presents the layout of the elements of the pressure control system in the orthosis without the safety valve, the fig. 3 presents the layout of the elements of the pressure control system in the orthosis without the safety valve together with the magnified control elements, and the fig. 4 presents the layout of the elements of the pressure control system in the orthosis with the safety valve together with the magnified control elements.

### Example I

The lower limb orthosis according to the invention consists of the rigid, non-deformable, bipartite posterior (1) and anterior (2) casing, a pneumatic cuff (3) located in the casing connected in a detachable way with the casing (1) and (2), as well as a pumping system (4) connected to the pneumatic cuff (3) with a hose together with a control module (10) which controls the functioning of the pumping system (4) and a pressure sensor (5). The control module (10) contains a microprocessor control unit connected to the pressure sensor (5) and the pumping system (4) and the pressure relief valve (6), and the pumping system is equipped with an electric pump. The pressure sensor (5) is a differential sensor with a range up to 75 mmHg. The pumping system (4) is located at the same printed board with the control module (10). From the control module (10) to the surface of the casing (2) runs a device signalling the exceeded maximum pressure; it has the form of a visual signalling device (diode) (7), and the pressure at which the signal is generated is 29 mmHg.

The non-deformable casing is bipartite and consists of the posterior part (1) enclosing the back part of the calf the bottom (sole) part of the foot and of the tibia - anterior part (2) enclosing tibia and the upper (dorsal) part of the foot. Both parts are mutually connected with self-attaching straps (8). The parts of the casing stabilise the pneumatic cuff (3) located between them.

The pneumatic cuff (3) is built of the layers forming the airtight compartment; the external surfaces of these layers are covered with soft fabric. The airtight compartment is equipped with the stem for connecting it to the pumping system (4) and with a pressure relief valve (6). The pneumatic cuff (3) is adapted to the anatomic shape of the joint and is equipped with an opening for the heel and Achilles tendon.

The method to control the pressure exerted by the orthosis on the limb consists in that when the limb is placed in the pneumatic cuff (3) of the ankle joint orthosis and closed in the non-deformable casing (1) and (2) with the self-attaching straps (8), the pumping system (4) power supply is switched on. The air is pumped by the pumping system (4) to the compartment of the pneumatic cuff via the pressure sensor in at least 10 sec cycles where each cycle is launched by the subsequent turning on of the power supply of the pumping system. During pumping the control module analyses the pressure in the compartment and when the pressure of 25 mmHg is exceeded, it emits a warning signal, and on reaching the pressure of 29 mmHg it disconnects the power supply of the pumping system and emits the continuous warning signal. If the pressure exceeding 29 mmHg is reached, the control module (10) opens the pressure relief valve (6) until the pressure drops to 25 mmHg. In case of any defect of the control unit (10) or the pressure sensor (5), on exceeding the pressure of 30 mmHg the safety valve (11) opens and reduces the pressure to at least 25 mmHg.

### Example II

The lower limb orthosis according to the invention consists of the rigid, non-deformable, bipartite posterior (1) and anterior (2) casing connected in a detachable way with the casing (1) and (2), a pneumatic cuff (3) located in the casing, and a pumping system (4) connected to the pneumatic cuff (3) with a hose together with a control module and a pressure sensor (5). The control module (10) contains a microprocessor control unit connected to the pressure sensor (5) and the pumping system (4) and the pressure relief valve (6), and the pumping system is equipped with a manual pump (4). The pressure sensor (5) is a differential sensor with a range up to 75 mmHg. From the control module (10) to the surface of the casing (2) runs a device signalling the exceeded maximum pressure; it has the form of a visual (diode) (7) and acoustic (buzzer) (9) signalling device, and the pressure at which the signal is generated is 29 mmHg.

The non-deformable casing is bipartite and consists of the posterior part (1) enclosing the back part of the calf the bottom (sole) part of the foot and of the tibia - anterior part (2) enclosing tibia and the upper (dorsal) part of the foot. Both parts are mutually connected with self-attaching straps (8). The parts of the casing stabilise the pneumatic cuff (3) located between them.

The pneumatic cuff (3) is built of the layers forming the airtight compartment, the external surface of these layers is covered with soft fabric. The airtight compartment is equipped with the stem for connecting it to the pumping system (4) and with a pressure relief valve (6). The pneumatic cuff (3) is adapted to the anatomic shape of the joint and is equipped with an opening for the heel and Achilles tendon.

The method to control the pressure exerted by the orthosis on the limb consists in that when the limb is placed in the pneumatic cuff (3) of the ankle joint orthosis and closed in the non-deformable casing (1) and (2) with the self-attaching straps (8), the power supply of the control module system (10) is switched on and the air is pumped with the manual pump to the compartment of the pneumatic cuff (3). The air is pumped by the pumping system (4) to the compartment of the pneumatic cuff (3) via the pressure sensor (5), the control module (10) works in at least 10 sec cycles where each cycle is launched by the subsequent turning on the power supply of the control module system (10). During pumping the control module (10) analyses the pressure in the compartment of the pneumatic cuff (3) and when the pressure of 25 mmHg is exceeded, it emits a warning signal, and on reaching the pressure of 29 mmHg it emits the continuous warning signal.

### Example III

The lower limb orthosis according to the invention consists of the rigid, non-deformable, bipartite posterior (1) and anterior (2) casing connected in a detachable way with the casing (1) and (2), a pneumatic cuff (3) located in the casing, a pumping system (4) connected to the pneumatic cuff (3) with a hose together with a control module and a pressure sensor (5). The control module (10) contains a microprocessor control unit connected to the pressure sensor (5) and the pumping system (4) and the pressure relief valve (6), and the pumping system is equipped with a manual pump (4). The pressure sensor (5) is a differential sensor with a range up to 75 mmHg. From the control module (10) to the surface of the casing (2) runs a device signalling the exceeded maximum pressure; it has the form of a visual (7) and acoustic (9) signalling device, and the pressure at which the signal is generated is 29 mmHg.

The non-deformable casing is bipartite and consists of the posterior part (1) enclosing the back part of the calf the bottom (sole) part of the foot and of the tibia - anterior part (2) enclosing tibia and the upper (dorsal) part of the foot. Both parts are mutually connected with self-attaching straps (8). The parts of the casing stabilise the pneumatic cuff (3) located between them.

The pneumatic cuff (3) is built of the layers forming the airtight compartment, the external surface of these layers is covered with soft fabric. The airtight compartment is equipped with the stem for connecting it to the pumping system (4) and with a pressure relief valve (6). The pneumatic cuff (3) is adapted to the anatomic shape of the joint and is equipped with an opening for the heel and Achilles tendon.

The method to control the pressure exerted by the orthosis on the limb consists in that when the limb is placed in the pneumatic cuff (3) of the ankle joint orthosis and closed in the non-deformable casing (1) and (2) with the self-attaching straps (8), the power supply of the control module system (10) is switched on and the air is pumped with the manual pump to the compartment of the pneumatic cuff (3). The air is pumped by the pumping system (4) to the compartment of the pneumatic cuff via the pressure sensor (5), and the control module (10) works in at least 10 sec cycles where each cycle is launched by the subsequent turning on the power supply of the control module system (10). During pumping the control module (10) analyses the pressure in the compartment of the pneumatic cuff (3) and when the pressure of 25 mmHg is exceeded, it emits a warning signal, and on reaching the pressure of 29 mmHg it emits the continuous warning signal.

If the pressure exceeding 29 mmHg is reached, the control module (10) opens the pressure relief valve (6) until the pressure drops to 25 mmHg. In case of any defect of the control unit (10) or the pressure sensor (5), on exceeding the pressure of 30 mmHg the safety valve (11) opens and reduces the pressure to at least 25 mmHg.

### List of reference numerals

- 1: - back casing
- 2: - frontcasing
- 3: - pneumatic cuff
- 4: - pumping system
- 5: - pressure sensor
- 6: - pressure relief valve
- 7: - diode
- 8: - self-attaching straps
- 9: - buzzer
- 10: - control module
- 11: - safety valve

## Claims

1. The lower limb orthosis containing the rigid, non-deformable, at least bipartite casing consisting of the posterior part enclosing the back part of the calf and the bottom part of the foot and of the tibia part enclosing tibia and the upper part of the foot, both parts connected with self-attaching straps, a pneumatic cuff located in the casing and connected in a detachable way with the casing, and a pumping system connected to the pneumatic cuff with a hose, **characterised in that** the pumping system (4) is connected with the control module (10) monitoring the functioning of the pumping system (4) and at least one pressure sensor (5).

2. Orthosis according to claim 1 **characterised in that** the pumping system (4) has the form of an electric pump.

3. Orthosis according to claim 1 **characterised in that** the pumping system (4) has the form of a manual pump.

4. Orthosis according to claim 1 or 2 or 3 **characterised in that** the control module (10) contains a microprocessor control unit connected to the pressure sensor (5) and the pumping system (4) and the pressure relief valve (6).

5. Orthosis according to claim 1 or 4 **characterised in that** the pressure sensor (5) is a differential sensor with a range at least up to 75 mmHg.

6. Orthosis according to claim 1 or 2 or 4 or 5 **characterised in that** the pumping system (4) with an electric pump is located at the same printed board as the microprocessor control unit.

7. Orthosis according to claim 1 or 2 or 3 or 4 or 5 or 6 **characterised in that** from the control module (10) to the surface of the casing runs at least one device signalling the exceeded maximum pressure.

8. Orthosis according to claim 7 **characterised in that** the signalling device has the form of at least the visual signalling device - a diode (7).

9. Orthosis according to claim 7 **characterised in that** he signalling device has the form of at least the visual signalling device - a diode (7) and the acoustic signalling device - a buzzer (9), and the pressure at which the visual and/or acoustic signal is generated does not exceed 30 mmHg.

10. Orthosis according to any of the previous claims **characterised in that** the pneumatic cuff (3) is built of at least two layers forming the airtight compartment, the external surface of these layers is covered with soft fabric.

11. Orthosis according to claim 10 **characterised in that** the airtight compartment is equipped with the stem for connecting it to the pumping system (4) and with a safety valve (11).

12. Orthosis according to any of the previous claims **characterised in that** the pneumatic cuff (3) is adapted to the anatomic shape of the joint and is equipped with at least an opening for the heel.

13. The method to control the pressure exerted by the orthosis on the limb **characterised in that** the limb is placed in the pneumatic cuff (3) of the ankle joint orthosis and closed in the non-deformable casing (1) and (2) with the self-attaching straps (8), after which the power supply of the pumping system (4) is switched on and the air is pumped by the pumping system (4) to the compartment of the pneumatic cuff (3) via the pressure sensor (5) in at least 10 sec cycles, and on exceeding the pressure of 25 mmHg it emits warning signal, and on reaching the pressure of 29 mmHg the control module disconnects the power supply of the pumping system emits the continuous warning signal.

14. The method according to claim 13 **characterised in that** each cycle of work of the pumping system (4) is launched by the subsequent turning on of the power supply of the pumping system (4).

15. The method according to claim 13 or 14 **characterised in that** in case of any defect of the control unit (10) or the pressure sensor (5), on exceeding the pressure of 30 mmHg the safety valve (11) opens and reduces the pressure to at least 25 mmHg.
